# EUROPÄISCHE PATENTANMELDUNG

(11) **EP 3 984 564 A1**
(43) Veröffentlichungstag der Anmeldung: **20.04.2022**
(21) Anmeldenummer: 21202943.3
(22) Anmeldetag: 15.10.2021
(51) Int. Cl.: A61L 2/26, A61B 50/30

(54) **STERILBEHÄLTERHALTEVORRICHTUNG FÜR EINEN STERILBEHÄLTER**

(30) Priorität: 16.10.2020 DE 102020127371
(71) Anmelder: Innovations Medical GmbH, 78532 Tuttlingen (DE)
(72) Erfinder: Kreidler, Jochen, 78532 Tuttlingen (DE)
(74) Vertreter: Daub, Thomas

(57) **Zusammenfassung**

Die Erfindung geht aus von einer Sterilbehälterhaltevorrichtung für einen Sterilbehälter (12), mit zumindest einem Grundkörper (14) zu einer festen Verbindung mit einem Sterilbehältergehäuse (16) des Sterilbehälters (12) und mit zumindest einem relativ zu dem Grundkörper (14) schwenkbar gelagerten Griffelement (18),

Es wird vorgeschlagen, dass die Sterilbehälterhaltevorrichtung zumindest ein mit dem zumindest einen Griffelement (18) gekoppeltes Rückstellelement (20, 20') aufweist, das dazu vorgesehen ist, das Griffelement (18) in zumindest einer Relativstellung zu dem Grundkörper (14) mit einer Kraft zu beaufschlagen.

## Beschreibung

### Stand der Technik

Die Erfindung betrifft eine Sterilbehälterhaltevorrichtung für einen Sterilbehälter.

Es ist bereits eine Sterilbehälterhaltevorrichtung für einen Sterilbehälter, mit zumindest einem Grundkörper zu einer festen Verbindung mit einem Sterilbehältergehäuse des Sterilbehälters und mit zumindest einem relativ zu dem Grundkörper schwenkbar gelagerten Griffelement vorgeschlagen worden.

Die Aufgabe der Erfindung besteht insbesondere darin, eine gattungsgemäße Sterilbehälterhaltevorrichtung und/oder einen gattungsgemäßen Sterilbehälter mit verbesserten Eigenschaften hinsichtlich eines Komforts und/oder einer Verstaubarkeit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Sterilbehälterhaltevorrichtung für einen Sterilbehälter, mit zumindest einem Grundkörper zu einer festen Verbindung mit einem Sterilbehältergehäuse des Sterilbehälters und mit zumindest einem relativ zu dem Grundkörper schwenkbar gelagerten Griffelement.

Es wird vorgeschlagen, dass die Sterilbehälterhaltevorrichtung zumindest ein mit dem zumindest einen Griffelement gekoppeltes Rückstellelement aufweist, das dazu vorgesehen ist, das Griffelement in zumindest einer Relativstellung zu dem Grundkörper mit einer Kraft zu beaufschlagen. Vorzugsweise ist das Rückstellelement dazu vorgesehen, das Griffelement in zumindest einer Relativstellung zu dem Grundkörper unabhängig von einer Ausrichtung der Sterilbehälterhaltevorrichtung mit einer Kraft zu beaufschlagen. Bevorzugt ist das Rückstellelement dazu vorgesehen, das Griffelement nach einer Auslenkung aus einer Ausgangslage heraus, wie insbesondere durch einen Bediener zu einer Nutzung der Sterilbehälterhaltevorrichtung, zurück in eine Ausgangslage zu bewegen. Vorzugsweise ist das Griffelement insbesondere von einem schwenkbaren Bügelgriff gebildet. Eine Schwenkachse des Griffelements verläuft insbesondere parallel zu einer Haupterstreckungsebene der Sterilbehälterhaltevorrichtung. Unter einer "Haupterstreckungsebene" einer Baueinheit soll insbesondere eine Ebene verstanden werden, welche parallel zu einer größten Seitenfläche eines kleinsten gedachten Quaders ist, welcher die Baueinheit gerade noch vollständig umschließt, und insbesondere durch den Mittelpunkt des Quaders verläuft. Das Griffelement weist insbesondere einen länglichen Griffbereich auf, wobei die Schwenkachse insbesondere auf einer gegenüberliegenden Seite des Griffelements, parallel zu dem Griffbereich des Griffelements, verläuft. Vorzugsweise ist das Rückstellelement mit einem Ende mit dem Griffelement verbunden und zu einer Abstützung an einem weiteren Ende mit dem Grundkörper der Sterilbehälterhaltevorrichtung verbunden. Das Rückstellelement ist insbesondere zumindest teilweise in einem Bereich der Schwenkachse des Griffelements angeordnet. Vorzugsweise ist das Rückstellelement zumindest teilweise koaxial zu der Schwenkachse angeordnet. Es wäre jedoch auch eine andere, einem Fachmann als sinnvoll erscheinende Anordnung des Rückstellelements denkbar. Unter einem "Rückstellelement" soll in diesem Zusammenhang insbesondere ein Element, vorzugsweise ein Federelement, verstanden werden, welches dazu vorgesehen ist, das Griffelement nach einer Verschwenkung in eine definierte Ausgangslage zurück zu bewegen. Vorzugsweise ist das Rückstellelement dazu vorgesehen, zumindest bei einer Auslenkung des Griffelements aus einer Ausgangslage, der Auslenkung eine Rückstellkraft entgegenzuwirken. Die Rückstellkraft ist insbesondere zumindest größer als eine Gewichtskraft des Griffelements. Das Rückstellelement ist insbesondere von einem elastischen Element, wie insbesondere einem Federelement, gebildet. Alternativ oder zusätzlich wäre auch denkbar, dass das Rückstellelement zumindest einen pneumatisches und/oder hydraulisches Element, wie insbesondere einen Kolben, aufweist, das dazu vorgesehen, zumindest bei einer Auslenkung des Griffelements aus einer Ausgangslage, der Auslenkung eine Rückstellkraft entgegenzuwirken. Es wäre jedoch auch denkbar, dass Rückstellelement von einem elektrischen Stellglied, wie insbesondere einem Elektromotor, gebildet, welcher das Griffelement nach einer Auslenkung des Griffelements aus einer Ausgangslage aktiv zurück in eine Ausgangslage bewegt. Dabei wäre insbesondere denkbar, dass in einer Schwenkachse des Griffelements eine Verzahnung ausgebildet ist, in welche ein Zahnrad des Rückstellelements eingreift. Zusätzlich könnte das Rückstellelement dabei zudem zu einer Energiegewinnung genutzt werden, wie insbesondere bei einer Auslenkung des Griffelements aus einer Ausgangslage. Alternativ oder zusätzlich wäre auch denkbar, dass das Rückstellelement ein Piezoelement umfasst, das beispielsweise bei einer Auslenkung des Griffelements aus einer Ausgangslage eine elektrische Energie erzeugt, wobei die Energie insbesondere dazu genutzt werden können, das Griffelement zurück in die Ausgangslage zu bewegen. Ferner soll dabei unter einem "Federelement" insbesondere ein makroskopisches Element verstanden werden, das zumindest eine Erstreckung und/oder eine relative Drehlage der Enden aufweist, die in einem normalen Betriebszustand um zumindest 10%, insbesondere um wenigstens 20%, vorzugsweise um mindestens 30% und besonders vorteilhaft um zumindest 50%, elastisch veränderbar ist, und das insbesondere eine von einer Veränderung der Erstreckung und/oder der relativen Drehlage abhängige und vorzugsweise zu der Veränderung proportionale Gegenkraft erzeugt, die der Veränderung entgegenwirkt. Unter einer "Erstreckung" eines Elements soll insbesondere ein maximaler Abstand zweier Punkte einer senkrechten Projektion des Elements auf eine Ebene verstanden werden. Unter einem "makroskopischen Element" soll insbesondere ein Element mit einer Erstreckung von zumindest 1 mm, insbesondere von wenigstens 5 mm und vorzugsweise von mindestens 10 mm, verstanden werden.

Der Grundkörper ist insbesondere von einer Grundplatte gebildet, welche zu einer direkten Befestigung an einem Sterilbehältergehäuse des Sterilbehälters vorgesehen ist. Vorzugsweise ist der Grundkörper insbesondere dazu vorgesehen, an einer Seitenwand des Sterilbehältergehäuses des Sterilbehälters befestigt zu werden. Eine Befestigung kann dabei beispielsweise durch Schweißen, Kleben, Nieten, Schauben und/oder eine andere, einem Fachmann als sinnvoll erscheinende Befestigungsmethode erfolgen. Es wäre insbesondere auch denkbar, dass der Grundkörper zumindest teilweise einstückig mit dem Sterilbehältergehäuse des Sterilbehälters ausgebildet ist. Unter einem "Grundkörper" soll in diesem Zusammenhang insbesondere ein Bauteil verstanden werden, welches eine tragende Struktur der Sterilbehälterhaltevorrichtung ausbildet. Vorzugsweise besteht der Grundkörper aus einem formfesten Material und erstreckt sich entlang einer Haupterstreckungsrichtung der Sterilbehälterhaltevorrichtung zumindest über einen Großteil der Sterilbehälterhaltevorrichtung. Bevorzugt ist der Grundkörper von einem einstückigen Bauteil gebildet, welches zu einer Lagerung und Fixierung des Griffelements und/oder eines Verschlusses vorgesehen ist. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Unter einer "Haupterstreckungsrichtung" eines Objekts soll dabei insbesondere eine Richtung verstanden werden, welche parallel zu einer längsten Kante eines kleinsten geometrischen Quaders verläuft, welcher das Objekt gerade noch vollständig umschließt. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung der Sterilbehälterhaltevorrichtung kann insbesondere vorteilhaft eine selbstständige Rückstellung des Griffelements erreicht werden. Es kann insbesondere erreicht werden, dass das Griffelement bei einer Lagerung und/oder einem Transport ohne Benutzung der Griffelemente immer in derselben Stellung bleibt. Hierdurch kann ein einfaches, immergleiches Greifen des Griffelements erreicht werden, wodurch ein hoher Komfort erreicht werden kann. Ferner kann ein Klappern des Griffelements vermieden werden, es kann insbesondere ein ständiges Anschlagen des Griffelements an dem Grundkörper oder einem Sterilbehältergehäuse des Sterilbehälters beispielsweise bei einem Transport ohne Benutzung der Griffelemente vermieden werden. Des Weiteren kann bei einem Reinigen und/oder Sterilisieren des Sterilbehälters, wie insbesondere durch Spülen, Autoklavieren oder dergleichen, ein Verklemmen des Griffelements vermieden werden. Gleichzeitig kann insbesondere eine kompakte immergleiche Anordnung des Griffelements bei der Reinigung und/oder einer Lagerung gewährleistet werden.

Ferner wird vorgeschlagen, dass das Rückstellelement dazu vorgesehen ist, das Griffelement mit einer Kraft in Richtung einer Ausgangslage des Griffelements zu beaufschlagen. Vorzugsweise ist das Rückstellelement dazu vorgesehen, das Griffelement mit einem Drehmoment in Richtung einer Ausgangslage des Griffelements zu beaufschlagen. Die Ausgangslage kann dabei insbesondere von einer Anschlagsposition gebildet sein, in welcher das Griffelement an einem Anschlag, wie insbesondere einem Anschlag der Grundkörpers, anliegt. Bevorzugt erstreckt sich eine Haupterstreckungsebene des Griffelements in der Ausgangslage zumindest im Wesentlichen parallel zu der Haupterstreckungsebene des Grundkörpers der Sterilbehälterhaltevorrichtung. Das Griffelement liegt in der Ausgangslage insbesondere zumindest teilweise an dem Grundkörper der Sterilbehälterhaltevorrichtung und/oder an dem Sterilbehältergehäuse des Sterilbehälters an. Insbesondere ist das Griffelement zu einem Greifen dazu vorgesehen, von dem Grundkörper der Sterilbehälterhaltevorrichtung weggeschwenkt zu werden. Unter "im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2°, aufweist. Dadurch ist das Griffelement insbesondere in Ruhe in einer definierten Lage angeordnet. Hierdurch kann insbesondere ein gezieltes Greifen des Griffelements ermöglicht werden.

Des Weiteren wird vorgeschlagen, dass das Rückstellelement als eine Feder ausgebildet ist. Das Rückstellelement ist insbesondere als eine Schraubenfeder ausgebildet. Es sind jedoch auch andere, einem Fachmann als sinnvoll erscheinende Ausgestaltungen des Rückschlagelements denkbar. Insbesondere wäre denkbar, dass das Rückschlagelement als eine Spiralfeder oder Blattfeder ausgebildet ist. Alternativ wäre jedoch auch denkbar, dass das Rückstellelement beispielsweise von einem Elastomerkörper gebildet ist, welcher zu einer Torsion vorgesehen ist. Eine Mittelachse der Feder, insbesondere der Schraubenfeder, erstreckt sich vorzugsweise koaxial zu einer Schwenkachse des Griffelements. Vorzugsweise sind insbesondere zwei Rückstellelemente vorgesehen, welche an beiden Enden des Griffelements angeordnet sind. Die Enden des Griffelements sind insbesondere jeweils in der Schwenkachse des Griffelements angeordnet. Dadurch kann insbesondere ein konstruktiv einfaches Rückstellelement bereitgestellt werden.

Ferner kann insbesondere ein vorteilhaft kompaktes Rückstellelement bereitgestellt werden.

Es wird ferner vorgeschlagen, dass die Sterilbehälterhaltevorrichtung zumindest eine Lagerausnehmung zur beweglichen Lagerung des Griffelements aufweist, welche zumindest im Wesentlichen von dem Grundkörper begrenzt ist. Vorzugsweise weist die Sterilbehälterhaltevorrichtung zumindest zwei Lagerausnehmungen zur beweglichen Lagerung des Griffelements auf, welche zumindest im Wesentlichen von dem Grundkörper begrenzt sind. Die zumindest eine Lagerausnehmung ist insbesondere zu einer den Griffelementen abgewandten Rückseite des Grundkörpers hin zumindest teilweise geöffnet ausgebildet. Es wäre jedoch auch denkbar, dass die Lagerausnehmung von einer sich parallel zu der Schwenkachse erstreckenden Bohrung in dem Grundkörper gebildet ist. Vorzugsweise ist die zumindest eine Lagerausnehmung insbesondere von einer Nut in dem Grundkörper gebildet, welche zu einer Rückseite des Grundkörpers hin geöffnet ist und welche über eine Öffnung in dem Grundkörper mit einer Vorderseite des Grundkörpers verbunden ist. Das Griffelement ist, vorzugsweise mit einem Ende, insbesondere durch die Öffnung des Grundkörpers in die Lagerausnehmung geführt. Die Lagerausnehmung dient insbesondere zu einer definierten schwenkbaren Lagerung des Griffelements relativ zu dem Grundkörper. Vorzugsweise ist ein Ende des Griffelements in der Lagerausnehmung in einer Ebene senkrecht zu der Schwenkachse zumindest zu einem wesentlichen Teil, insbesondere in einem Winkelbereich von zumindest 120°, vorzugsweise von zumindest 180° und besonders bevorzugt von zumindest 240°, von der Lagerausnehmung umgeben. Vorzugsweise ist die Lagerausnehmung insbesondere dazu vorgesehen, ein Ende des Griffelements, insbesondere senkrecht zu der Schwenkachse, zumindest teilweise zu umgreifen. Dadurch kann insbesondere eine vorteilhafte Lagerung des Griffelements bereitgestellt werden. Es kann insbesondere auf zusätzliche Lagerbauteile verzichtet werden.

Es wird weiter vorgeschlagen, dass das Rückstellelement zumindest im Wesentlichen in der Lagerausnehmung angeordnet ist. Vorzugsweise ist das Rückstellelement in der Lagerausnehmung in einer Ebene senkrecht zu der Schwenkachse zumindest zu einem wesentlichen Teil, insbesondere in einem Winkelbereich von zumindest 120°, vorzugsweise von zumindest 180° und besonders bevorzugt von zumindest 240°, von der Lagerausnehmung umgeben. Vorzugsweise ist die Lagerausnehmung insbesondere dazu vorgesehen, das Rückstellelement, insbesondere senkrecht zu der Schwenkachse, zumindest teilweise zu umgreifen. Vorzugsweise weist die Lagerausnehmung eine zumindest annähernd zylindrische Grundform auf. Vorzugsweise ist entlang eines axialen Verlaufs der Lagerausnehmung in der Lagerausnehmung das Rückstellelement und darauffolgend das Ende des Griffelements angeordnet. Dadurch kann insbesondere eine vorteilhaft kompakte Anordnung des Rückstellelements bereitgestellt werden. Dadurch kann insbesondere eine vorteilhaft kompakte Sterilbehälterhaltevorrichtung bereitgestellt werden.

Zudem wird vorgeschlagen, dass eine Mittelachse des Rückstellelements zumindest im Wesentlichen parallel zu einer Schwenkachse des Griffelements angeordnet ist. Vorzugsweise ist die Mittelachse des Rückstellelements zumindest im Wesentlichen koaxial zu der Schwenkachse des Griffelements angeordnet. Die Mittelachse des Rückstellelements verläuft insbesondere parallel zu einer Haupterstreckungsrichtung des Rückstellelements. Die Mittelachse des Rückstellelements ist insbesondere von einer Torsionsachse des Rückstellelements gebildet. Dadurch kann insbesondere eine vorteilhaft kompakte Anordnung des Rückstellelements bereitgestellt werden. Dadurch kann insbesondere eine vorteilhaft kompakte Sterilbehälterhaltevorrichtung bereitgestellt werden.

Ferner wird vorgeschlagen, dass das Rückstellelement zumindest einen ersten Endfortsatz, welcher dazu vorgesehen ist, in eine Ausnehmung des Griffelements einzugreifen, und zumindest einen zweiten Endfortsatz aufweist, welcher dazu vorgesehen ist, in eine Ausnehmung des Grundkörpers einzugreifen. Vorzugsweise ist der erste Endfortsatz dazu vorgesehen, ein erstes Ende des Rückstellelements drehfest mit dem Griffelement zu verbinden. Bevorzugt ist der zweite Endfortsatz dazu vorgesehen, ein zweites Ende des Rückstellelements drehfest mit dem Grundkörper zu verbinden. Zwischen dem ersten Ende des Rückstellelements und dem zweiten Ende des Rückstellelements kann zu einer Erzeugung einer Rückstellkraft das Rückstellelement verdreht, insbesondere tordiert, werden. Der erste Endfortsatz und/oder der zweite Endfortsatz ist/sind insbesondere jeweils von einem Drahtende des als Feder ausgebildeten Rückstellelements ausgebildet. Es wäre jedoch auch eine andere, einem Fachmann als sinnvoll erscheinende Ausgestaltung der Endfortsätze denkbar. Je nach Ausgestaltung des Griffelements und/oder des Grundkörpers wäre auch denkbar, dass der erste Endfortsatz von dem zweiten Endfortsatz verschieden ausgebildet ist. Dadurch kann insbesondere eine vorteilhafte Montage des Rückstellelements erreicht werden. Ferner kann insbesondere eine vorteilhaft direkte Kraftübertragung erreicht werden. Zudem kann insbesondere eine direkte Abstützung des Rückstellelements an dem Grundkörper erreicht werden.

Des Weiteren schlägt die Erfindung einen Sterilbehälter mit zumindest einem Sterilbehältergehäuse, und mit der zumindest einen an dem Sterilbehältergehäuse angeordneten Sterilbehälterhaltevorrichtung, vor. Der Sterilbehälter ist bevorzugt als medizinischer Sterilbehälter ausgebildet. Der Sterilbehälter umfasst vorzugsweise einen Behälterdeckel und das Sterilbehältergehäuse. Das Sterilbehältergehäuse ist vorzugsweise mittels des Behälterdeckels auf eine, einem Fachmann bereits bekannte Art und Weise, insbesondere hermetisch, verschließbar. Vorzugsweise weist der Sterilbehälter dazu insbesondere zumindest eine, insbesondere zumindest zwei, einander gegenüberliegende Verschlusseinheiten auf. Die Verschlusseinheit weist insbesondere zumindest einen Spannhebel auf.

Die Sterilbehälterhaltevorrichtung ist mittels des Grundkörpers der Sterilbehälterhaltevorrichtung an dem Sterilbehältergehäuse angeordnet. Der Grundkörper der Sterilbehälterhaltevorrichtung ist insbesondere von einer Grundplatte gebildet, welche direkt an dem Sterilbehältergehäuse des Sterilbehälters befestigt ist. Vorzugsweise ist der Grundkörper insbesondere dazu vorgesehen, an einer Seitenwand des Sterilbehältergehäuses des Sterilbehälters befestigt zu werden. Eine Befestigung kann dabei beispielsweise durch Schweißen, Kleben, Nieten, Schauben und/oder eine andere, einem Fachmann als sinnvoll erscheinende Befestigungsmethode erfolgen. Mittels der erfindungsgemäßen Ausgestaltung des Sterilbehälters kann vorteilhaft ein hoher Bedienkomfort realisiert werden. Es kann insbesondere ein Sterilbehälter mit einem selbstständig rückstellenden Griffelement bereitgestellt werden. Hierdurch kann ein einfaches, immergleiches Greifen des Griffelements erreicht werden, wodurch ein hoher Komfort erreicht werden kann. Ferner kann ein Klappern des Griffelements vermieden werden, es kann insbesondere ein ständiges Anschlagen des Griffelements an dem Grundkörper oder einem Sterilbehältergehäuse des Sterilbehälters beispielsweise bei einem Transport ohne Benutzung der Griffelemente vermieden werden. Des Weiteren kann bei einem Reinigen und/oder Sterilisieren des Sterilbehälters, wie insbesondere durch Spülen, Autoklavieren oder dergleichen, ein Verklemmen des Griffelements vermieden werden. Gleichzeitig kann insbesondere eine kompakte immergleiche Anordnung des Griffelements bei der Reinigung und/oder einer Lagerung gewährleistet werden.

Es wird ferner vorgeschlagen, dass das Griffelement der Sterilbehälterhaltevorrichtung zumindest einen Griffbereich aufweist, der in einer Ausgangslage des Griffelements an dem Sterilbehältergehäuse anliegt. Der Griffbereich ist insbesondere dazu vorgesehen, direkt von einer Hand eines Benutzers umgriffen zu werden. Der Griffbereich ist insbesondere von einem Bereich des Griffelements gebildet, welcher zu einem direkten Kontaktieren durch den Benutzer vorgesehen ist. Der Griffbereich weist insbesondere eine längliche, vorzugsweise zylindrische, Grundform auf. Eine Schwenkachse des Griffelements verläuft insbesondere auf einer gegenüberliegenden Seite des Griffelements parallel zu dem Griffbereich des Griffelements. Vorzugsweise ist das Griffelement insbesondere U-förmig ausgebildet, wobei der Griffbereich in einem Mittelbereich des Griffelements angeordnet ist. Das Sterilbehältergehäuse bildet insbesondere zumindest teilweise einen Anschlag für das Griffelement aus, an welchem das Griffelement, insbesondere der Griffbereich des Griffelements, in der Ausgangslage anliegt. Dadurch kann insbesondere eine vorteilhaft kompakte Verstaustellung des Griffelements an dem Sterilbehältergehäuse bereitgestellt werden. Vorzugsweise erstreckt sich eine Haupterstreckungsebene des Griffelements in der Ausgangslage zumindest im Wesentlichen parallel zu einer Seitenwand des Sterilbehältergehäuses, an welcher die Sterilbehälterhaltevorrichtung angeordnet ist. Das Griffelement liegt dadurch in der Ausgangslage insbesondere flach an der Seitenwand des Sterilbehältergehäuses an. Hierdurch kann insbesondere eine vorteilhaft kompakte Form der Sterilbehälterhaltevorrichtung an dem Sterilbehältergehäuse bereitgestellt werden.

Es wird weiter vorgeschlagen, dass das Griffelement in einer Ausgangslage des Griffelements in dem Sterilbehältergehäuse eingebettet ist. Vorzugsweise ist in einer Außenwand, insbesondere der Seitenwand, an welcher die Sterilbehälterhaltevorrichtung angeordnet ist, eine Vertiefung für das Griffelement ausgeformt. Bevorzugt kann das Griffelement in der Ausgangslage zumindest teilweise in einer Vertiefung des Sterilbehältergehäuses versenkt ausgebildet sein. Die Vertiefung kann dabei beispielsweise als eine eingebrachte Nut ausgebildet sein als auch von einer tiefgezogenen Mulde gebildet sein. Es sind verschiedene, einem Fachmann als sinnvoll erscheinende Ausgestaltungen der Vertiefung denkbar. Dadurch kann insbesondere eine vorteilhaft kompakte Verstaustellung des Griffelements an dem Sterilbehältergehäuse bereitgestellt werden. Hierdurch kann insbesondere eine vorteilhaft kompakte Form der Sterilbehälterhaltevorrichtung an dem Sterilbehältergehäuse bereitgestellt werden.

Ferner wird vorgeschlagen, dass das Rückstellelement zumindest im Wesentlichen vollständig von dem Sterilbehältergehäuse und dem Grundkörper der Sterilbehälterhaltevorrichtung umschlossen ist. Vorzugsweise ist das Rückstellelement zumindest im Wesentlichen in der Lagerausnehmung angeordnet, wobei eine offene Seite der Lagerausnehmung wiederum durch das Sterilbehältergehäuse begrenzt ist. Vorzugsweise ist das Rückstellelement in der Lagerausnehmung in einer Ebene senkrecht zu der Schwenkachse zumindest zu einem wesentlichen Teil, insbesondere in einem Winkelbereich von zumindest 120°, vorzugsweise von zumindest 180° und besonders bevorzugt von zumindest 240°, von der Lagerausnehmung umgeben. Ferner ist das Rückstellelement in der Ebene insbesondere zu einer Seite hin von dem Sterilbehältergehäuse begrenzt. Vorzugsweise weist die Lagerausnehmung eine zumindest annähernd zylindrische Grundform auf, wobei eine Seite der Mantelfläche zu dem Sterilbehältergehäuse hin geöffnet ist. Dadurch kann insbesondere eine vorteilhaft kompakte Anordnung des Rückstellelements bereitgestellt werden. Dadurch kann insbesondere ein vorteilhaft kompakter Sterilbehälter bereitgestellt werden. Ferner kann dadurch insbesondere ein vorteilhaft einfaches Einbringen des Rückstellelements erreicht werden. Insbesondere kann bei einer Montage das Rückstellelement in die Lagerausnehmung eingelegt und anschließend die Sterilbehälterhaltevorrichtung an dem Sterilbehältergehäuse angeordnet werden. Es wäre jedoch auch denkbar, dass das Rückstellelement über eine seitliche Ausnehmung des Grundkörpers der Sterilbehälterhaltevorrichtung in die Lagerausnehmung eingebracht wird.

Des Weiteren wird vorgeschlagen, dass der Sterilbehälter eine weitere Sterilbehälterhaltevorrichtung aufweist, wobei die Sterilbehälterhaltevorrichtung und die weitere Sterilbehälterhaltevorrichtung an voneinander abgewandten Seiten des Sterilbehältergehäuses angeordnet sind. Vorzugsweise sind die Sterilbehälterhaltevorrichtung und die weitere Sterilbehälterhaltevorrichtung identisch ausgebildet. Bevorzugt sind die Sterilbehälterhaltevorrichtung und die weitere Sterilbehälterhaltevorrichtung jeweils an voneinander abgewandten Außenseiten von gegenüberliegenden Seitenwänden des Sterilbehältergehäuses angeordnet. Dadurch kann insbesondere ein vorteilhaft einfaches, beidseitiges Halten des Sterilbehälters ermöglicht werden.

Die erfindungsgemäße Sterilbehälterhaltevorrichtung und/oder der erfindungsgemäße Sterilbehälter sollen/soll hierbei nicht auf die oben beschriebene Anwendung und Ausführungsform beschränkt sein. Insbesondere können/kann die erfindungsgemäße Sterilbehälterhaltevorrichtung und/oder der erfindungsgemäße Sterilbehälter zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: einen erfindungsgemäßen Sterilbehälter mit einer erfindungsgemäßen Sterilbehälterhaltevorrichtung, mit einer weiteren erfindungsgemäßen Sterilbehälterhaltevorrichtung und mit einer Verschlussvorrichtung in einer schematischen Darstellung,

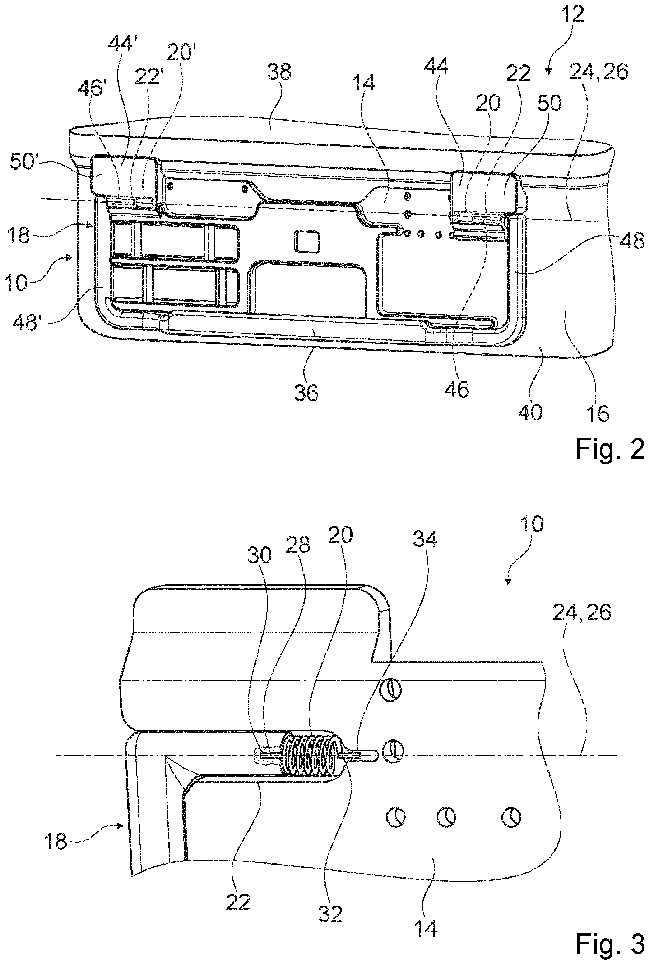

- Fig. 2: einen Teilausschnitt des erfindungsgemäßen Sterilbehälters mit der erfindungsgemäßen Sterilbehälterhaltevorrichtung in einer schematischen Darstellung, wobei die Verschlussvorrichtung ausgebildet ist,
- Fig. 3: einen Teilausschnitt der erfindungsgemäßen Sterilbehälterhaltevorrichtung mit einem Grundkörper, mit einem Griffelement und mit einem Rückstellelement in einem von dem Sterilbehälter demontierten Zustand in einer schematischen Rückansicht und

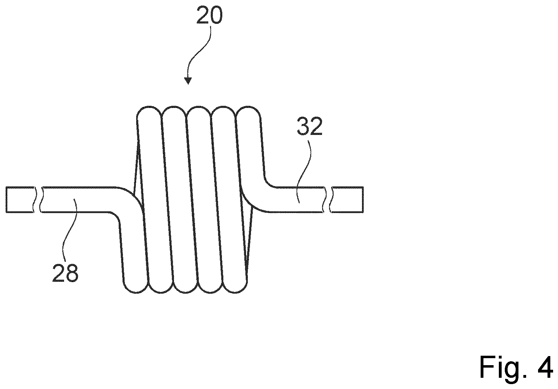

- Fig. 4: das Rückstellelement der erfindungsgemäßen Sterilbehälterhaltevorrichtung in einer schematischen Darstellung.

### Beschreibung des Ausführungsbeispiels

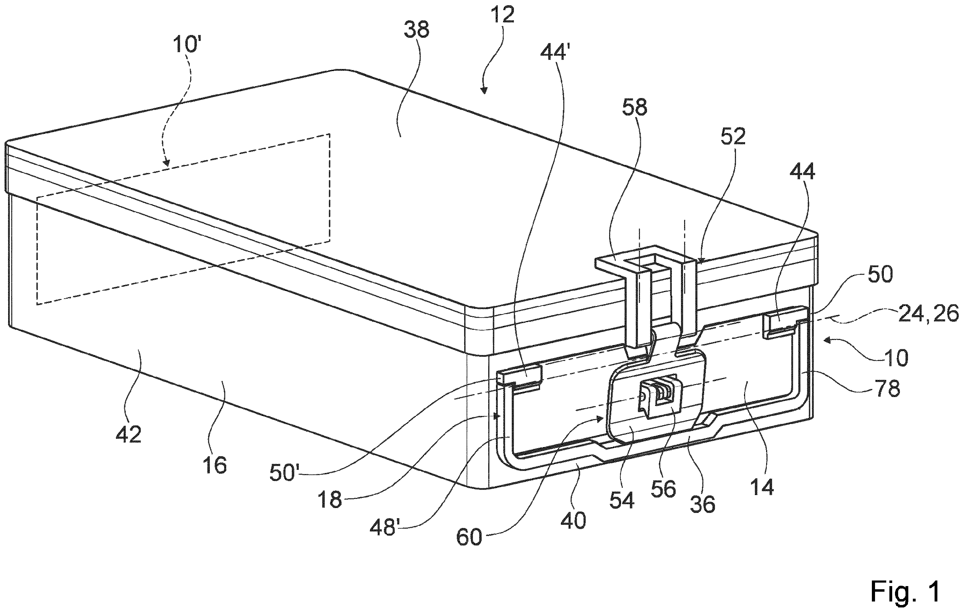

Figur 1 zeigt einen Sterilbehälter 12 mit zumindest einer Sterilbehälterhaltevorrichtung 10, 10'. Der Sterilbehälter 12 ist als medizinischer Sterilbehälter ausgebildet. Es ist jedoch auch denkbar, dass der Sterilbehälter 12 zu einer Verwendung in einem anderen Gebiet vorgesehen ist, in der eine sterile Aufbewahrung von Bauteilen und/oder Werkzeugen sinnvoll oder gewünscht ist. Der Sterilbehälter 12 umfasst einen Behälterdeckel 38 und ein Sterilbehältergehäuse 16. Das Sterilbehältergehäuse 16 ist vorzugsweise kastenartig ausgebildet und begrenzt einen Aufnahmeraum. Das Sterilbehältergehäuse 16 ist mittels des Behälterdeckels 38 auf eine, einem Fachmann bereits bekannte Art und Weise, insbesondere hermetisch, verschließbar. Das Sterilbehältergehäuse 16 ist zur Bildung eines geschlossenen Sterilraums mittels des Behälterdeckels 38 verschließbar. Der Behälterdeckel 38 ist abnehmbar an dem Sterilbehältergehäuse 16 angeordnet und mittels umlaufend zwischen dem Behälterdeckel 38 und dem Sterilbehältergehäuse 16 angeordneten Dichtungsmitteln luftdicht auf das Sterilbehältergehäuse 16 aufsetzbar und mit diesem verbindbar. Das Sterilbehältergehäuse 16 weist einen Boden und vier damit verbundene Seitenwände 40, 42 auf. Zwei der Seitenwände 40 bilden jeweils Stirnseiten des Sterilbehältergehäuses 16 aus.

Ferner weist der Sterilbehälter 12 zumindest eine an dem Sterilbehältergehäuse 16 angeordnete Sterilbehälterhaltevorrichtung 10, 10'. Der Sterilbehälter 12 weist eine Sterilbehälterhaltevorrichtung 10 und eine weitere Sterilbehälterhaltevorrichtung 10' auf. Die Sterilbehälterhaltevorrichtung 10 und die weitere Sterilbehälterhaltevorrichtung 10' sind an voneinander abgewandten Seiten des Sterilbehältergehäuses 16 angeordnet. Die Sterilbehälterhaltevorrichtung 10 und die weitere Sterilbehälterhaltevorrichtung 10' sind jeweils an voneinander abgewandten Außenseiten von gegenüberliegenden Seitenwänden 40 des Sterilbehältergehäuses 16 angeordnet. Die Sterilbehälterhaltevorrichtungen 10, 10' sind an den stirnseitigen Seitenwänden 40 angeordnet. Es wäre jedoch auch eine andere, einem Fachmann als sinnvoll erscheinende Anordnung denkbar. Die Sterilbehälterhaltevorrichtung 10 und die weitere Sterilbehälterhaltevorrichtung 10' sind identisch ausgebildet. Beschreibungen, die zu der Sterilbehälterhaltevorrichtung 10 gemacht werden, sind daher auch auf die weitere Sterilbehälterhaltevorrichtung 10' anwendbar. Zu einer Vereinfachung wird im Folgenden lediglich die Sterilbehälterhaltevorrichtung 10 beschrieben.

Die Sterilbehälterhaltevorrichtung 10 weist einen Grundkörper 14 zu einer festen Verbindung mit dem Sterilbehältergehäuse 16 des Sterilbehälters 12 auf. Der Grundkörper 14 ist von einer Grundplatte gebildet, welche zu einer direkten Befestigung an dem Sterilbehältergehäuse 16 des Sterilbehälters 12 vorgesehen ist. Der Grundkörper 14 weist eine plattenförmige Grundform auf. Der Grundkörper 14 ist zumindest im Wesentlichen quaderförmig. Der Grundkörper 14 besteht aus einem Metall. Es wäre jedoch auch eine andere, einem Fachmann als sinnvoll erscheinende Materialgebung denkbar. Der Grundkörper 14 ist an der Seitenwand 40 des Sterilbehältergehäuses 16 befestigt. Eine Befestigung kann dabei beispielsweise durch Schweißen, Kleben, Nieten, Schauben und/oder eine andere, einem Fachmann als sinnvoll erscheinende Befestigungsmethode erfolgen. Der Grundkörper 14 weist an den beiden äußeren Enden zwei Befestigungsfortsätze 44, 44' auf. Die Befestigungsfortsätze 44, 44' sind von zumindest annähernd quaderförmigen Fortsätzen gebildet, welche über eine plattenförmige Grundform des Grundkörpers 14 hinausragen.

Die Sterilbehälterhaltevorrichtung 10 weist ferner zumindest eine Lagerausnehmung 22, 22' auf. Die Sterilbehälterhaltevorrichtung 10 weist zwei Lagerausnehmungen 22, 22' auf. Die Lagerausnehmungen 22, 22' sind von dem Grundkörper 14 begrenzt. Die Lagerausnehmungen 22, 22' sind von den Befestigungsfortsätzen 44, 44' des Grundkörpers 14 begrenzt. Die Lagerausnehmungen 22, 22' weisen eine zumindest annähernd zylindrische Grundform auf. Die Lagerausnehmungen 22, 22' sind zu einer der Seitenwand 40 zugewandten Rückseite des Grundkörpers 14 hin zumindest teilweise geöffnet ausgebildet. Die Lagerausnehmungen 22, 22' sind jeweils von einer Nut in den Befestigungsfortsätzen 44, 44' gebildet, welche zu einer Rückseite des Grundkörpers 14 hin geöffnet sind. Die Lagerausnehmungen 22, 22' erstrecken sich bis zu einer Seitenkante des Grundkörpers 14 und sind jeweils zu einer Außenseite des Grundkörpers 14 hin geöffnet. Die Lagerausnehmungen 22, 22' erstrecken sich jeweils koaxial zueinander.

Ferner weist die Sterilbehälterhaltevorrichtung 10 ein relativ zu dem Grundkörper 14 schwenkbar gelagertes Griffelement 18 auf. Das Griffelement 18 ist von einem schwenkbaren Bügelgriff gebildet. Das Griffelement 18 ist von einem schwenkbaren, U-förmigen Bügelgriff gebildet. Das Griffelement 18 weist eine definierte Schwenkachse 26 auf. Die Schwenkachse 26 des Griffelements 18 verläuft parallel zu einer Haupterstreckungsebene der Sterilbehälterhaltevorrichtung 10. Die Schwenkachse 26 des Griffelements 18 verläuft parallel zu einer Haupterstreckungsebene des Sterilbehälters 12 und senkrecht zu einer Haupterstreckungsrichtung des Sterilbehälters 12. Das Griffelement 18 weist einen länglichen Griffbereich 36 auf, wobei die Schwenkachse 26 auf einer dem Griffbereich 36 gegenüberliegenden Seite des Griffelements 18, parallel zu dem Griffbereich 36 des Griffelements 18, verläuft. Enden 46, 46' des Griffelements 18 sind jeweils in der Schwenkachse 26 des Griffelements 18 angeordnet. Das Griffelement 18 erstreckt sich bügelförmig von einem ersten Ende 46, über einen ersten Kragbereich 48 über den Griffbereich 36, über einen zweiten Kragbereich 48' hin zu einem zweiten Ende 46'. Die Kragbereiche 48, 48' verlaufen jeweils senkrecht zu dem Griffbereich 36 und den Enden 46, 46'. Die Enden 46, 46' verlaufen in der Schwenkachse 26. Der Griffbereich 36 weist beispielhaft gegenüber den Kragbereichen 48, 48' des Griffelements 18 zu einem leichteren Greifen einen vergrößerten Querschnitt auf. Das Griffelement 18 weist in den Kragbereichen 48, 48' und in dem Griffbereich 36 beispielhaft einen ovalen Querschnitt auf. Das Griffelement 18 weist an den Enden 46, 46' jeweils einen runden Querschnitt auf. Der Griffbereich 36 ist von einem Bereich des Griffelements 18 gebildet, welcher zu einem direkten Kontaktieren durch den Benutzer vorgesehen ist. Der Griffbereich 36 weist eine längliche Grundform auf. Eine Schwenkachse 26 des Griffelements 18 verläuft auf einer dem Griffbereich 36 gegenüberliegenden Seite des Griffelements 18 parallel zu dem Griffbereich 36 des Griffelements 18.

Die Lagerausnehmungen 22, 22' sind zu einer beweglichen Lagerung des Griffelements 18 vorgesehen. Die Lagerausnehmungen 22, 22' sind zu einer schwenkbaren Lagerung des Griffelements 18 vorgesehen. Das Griffelement 18 ist mit den Enden 46, 46' jeweils in einer der Lagerausnehmungen 22, 22' gelagert. Das Griffelement 18 ist mit den Enden 46, 46' jeweils über eine äußere Öffnung des Grundkörpers 14 in die Lagerausnehmungen 22, 22' geführt. Die Lagerausnehmungen 22, 22' dienen insbesondere zu einer definierten schwenkbaren Lagerung des Griffelements 18 relativ zu dem Grundkörper 14. Die Enden 46, 46' des Griffelements 18 greifen von außen in die Befestigungsfortsätze 44, 44' ein. Die Enden 46, 46' des Griffelements 18 erstrecken sich von den Kragbereichen 48, 48' jeweils senkrecht nach innen in die Lagerausnehmungen 22, 22' hinein.

Des Weiteren weist die Sterilbehälterhaltevorrichtung 10 zumindest ein mit dem Griffelement 18 gekoppeltes Rückstellelement 20, 20' auf. Die Sterilbehälterhaltevorrichtung 10 weist beispielhaft zwei mit dem Griffelement 18 gekoppelte Rückstellelemente 20, 20' auf. Die Rückstellelemente 20, 20' sind dazu vorgesehen, das Griffelement 18 in zumindest einer Relativstellung zu dem Grundkörper 14 mit einer Kraft zu beaufschlagen. Die Rückstellelemente 20, 20' sind jeweils identisch ausgebildet.

Die Rückstellelemente 20, 20' sind dazu vorgesehen, das Griffelement 18 nach einer Auslenkung aus einer Ausgangslage heraus, wie insbesondere durch einen Bediener zu einer Nutzung der Sterilbehälterhaltevorrichtung 10, zurück in eine Ausgangslage zu bewegen. Die Rückstellelemente 20, 20' sind jeweils mit einem Ende 46, 46' des Griffelements 18 verbunden und zu einer Abstützung an einem weiteren Ende mit dem Grundkörper 14 der Sterilbehälterhaltevorrichtung 10 verbunden. Die Rückstellelemente 20, 20' sind dazu vorgesehen, das Griffelement 18 mit einer Kraft in Richtung der Ausgangslage des Griffelements 18 zu beaufschlagen. Die Rückstellelemente 20, 20' sind dazu vorgesehen, das Griffelement 18 mit einem Drehmoment in Richtung der Ausgangslage des Griffelements 18 zu beaufschlagen. Die Ausgangslage ist dabei von einer Anschlagsposition des Griffelements 18 gebildet sein, in welcher das Griffelement 18 an einem Anschlag anliegt. Eine Haupterstreckungsebene des Griffelements 18 erstreckt sich in der Ausgangslage im Wesentlichen parallel zu der Haupterstreckungsebene des Grundkörpers 14 der Sterilbehälterhaltevorrichtung 10. Das Griffelement 18 liegt in der Ausgangslage an dem Sterilbehältergehäuse 16 an. Der Griffbereich 36 des Griffelements 18 liegt in der Ausgangslage des Griffelements 18 teilweise an dem Sterilbehältergehäuse 16 an. Die Kragbereiche 48, 48' des Griffelements 18 liegen in der Ausgangslage des Griffelements 18 vollständig an dem Sterilbehältergehäuse 16 an. Zudem wäre denkbar, dass das Griffelement 18 in der Ausgangslage des Griffelements 18 zudem zumindest teilweise in dem Sterilbehältergehäuse 16 eingebettet ist. Dabei wäre insbesondere denkbar, dass in der Seitenwand 40, an welcher die Sterilbehälterhaltevorrichtung 10 angeordnet ist, eine Vertiefung für das Griffelement 18 ausgeformt ist. Die Vertiefung kann dabei beispielsweise als eine eingebrachte Nut ausgebildet sein als auch von einer tiefgezogenen Mulde gebildet sein.

Das Griffelement 18 ist zu einem Greifen dazu vorgesehen, von dem Grundkörper 14 der Sterilbehälterhaltevorrichtung 10 und der Seitenwand 40 des Sterilbehältergehäuses 16 weggeschwenkt zu werden. Zu einem leichteren Greifen des Griffelements 18 aus der Ausgangslage bildet der Griffbereich 36 des Griffelements 18 in einem Mittelbereich eine Bogen- und/oder Brückenform aus, wobei der Griffbereich 36 in dem Mittelbereich in der Ausgangslage zumindest teilweise von der Seitenwand 40 des Sterilbehältergehäuses 16 abgehoben ist.

Die Rückstellelemente 20, 20' sind jeweils als Feder ausgebildet. Die Rückstellelemente 20, 20' sind jeweils als Schraubenfeder ausgebildet. Es wäre jedoch auch eine andere, einem Fachmann als sinnvoll erscheinende Ausbildung der Rückstellelemente 20, 20' denkbar. Die Rückstellelemente 20, 20' sind in einem Bereich der Schwenkachse 26 des Griffelements 18 angeordnet. Die Rückstellelemente 20, 20' sind koaxial zu der Schwenkachse 26 angeordnet. Eine Mittelachse 24 der Rückstellelemente 20, 20' ist jeweils parallel zu der Schwenkachse 26 des Griffelements 18 angeordnet. Die Rückstellelemente 20, 20' sind jeweils im Wesentlichen in einer der Lagerausnehmungen 22, 22' angeordnet. Die Rückstellelemente 20, 20' sind jeweils in verschiedenen Lagerausnehmungen 22, 22' angeordnet. Die Rückstellelemente 20, 20' weisen jeweils einen ersten Endfortsatz 28, welcher dazu vorgesehen ist, in eine Ausnehmung 30 des Griffelements 18 einzugreifen, und jeweils einen zweiten Endfortsatz 32 auf, welcher dazu vorgesehen ist, in eine Ausnehmung 34 des Grundkörpers 14 einzugreifen. Der erste Endfortsatz 28 der Rückstellelemente 20, 20' ist jeweils dazu vorgesehen, ein erstes Ende des jeweiligen Rückstellelements 20, 20' drehfest mit einem der Enden 46, 46' des Griffelements 18 zu verbinden. Der zweite Endfortsatz 32 der Rückstellelemente 20, 20' ist dazu vorgesehen, ein zweites Ende des jeweiligen Rückstellelements 20, 20' drehfest mit dem Grundkörper 14 zu verbinden. Die Ausnehmungen 30 des Griffelements 18 sind jeweils an den Stirnseiten der Enden 46, 46' des Griffelements 18 angeordnet. Die Ausnehmungen 30 des Griffelements 18 sind exzentrisch angeordnet. Die Ausnehmungen 30 des Griffelements 18 sind zu der Schwenkachse 26 versetzt angeordnet. Die Ausnehmungen 30 sind beispielhaft von Bohrungen gebildet. Die Ausnehmungen 34 des Grundkörpers 14 sind jeweils an eine der Lagerausnehmung 22 angrenzend in einer die jeweilige Lagerausnehmung 22 begrenzenden Endwand des Grundkörpers 14 angeordnet. Die Ausnehmungen 34 des Grundkörpers 14 sind exzentrisch angeordnet. Die Ausnehmungen 34 des Grundkörpers 14 sind zu der Schwenkachse 26 versetzt angeordnet. Zwischen dem ersten Ende des jeweiligen Rückstellelements 20, 20' und dem zweiten Ende des jeweiligen Rückstellelements 20, 20' kann zu einer Erzeugung einer Rückstellkraft das jeweilige Rückstellelement 20, 20' verdreht, insbesondere tordiert, werden. Die Rückstellelemente 20, 20' werden insbesondere synchron verformt. Die ersten Endfortsätze 28 und die zweiten Endfortsätze 32 sind insbesondere jeweils von einem Drahtende des jeweiligen als Feder ausgebildeten Rückstellelements 20, 20' ausgebildet. Es wäre jedoch auch eine andere, einem Fachmann als sinnvoll erscheinende Ausgestaltung der Endfortsätze 28, 32 denkbar.

Die Rückstellelemente 20, 20' sind jeweils im Wesentlichen vollständig von dem Sterilbehältergehäuse 16 und dem Grundkörper 14 der Sterilbehälterhaltevorrichtung 10, 10' umschlossen. Die Rückstellelemente 20, 20' sind im Wesentlichen in einer der Lagerausnehmungen 22, 22' angeordnet, wobei eine erste offene Seite der jeweiligen Lagerausnehmung 22, 22' durch die Seitenwand 40 des Sterilbehältergehäuses 16 begrenzt ist und eine weitere offene Seite der jeweiligen Lagerausnehmung 22, 22' durch eines der Enden 46, 46' des Griffelements 18 geschlossen ist. Die Lagerausnehmungen 22, 22' sind jeweils durch eines der Rückstellelemente 20, 20' sowie eines der Enden 46, 46' des Griffelements 18 gefüllt. Die Rückstellelemente 20, 20' sind entlang der Schwenkachse 26 zwischen jeweils einem der Enden 46, 46' des Griffelements 18 und dem Grundkörper 14 angeordnet.

Die Befestigungsfortsätze 44, 44' weisen ferner jeweils einen Anschlag 50, 50' für das Griffelement 18 auf. Die Anschläge 50, 50' ragen jeweils parallel zu der Schwenkachse 26 nach außen aus einer Grundform der Befestigungsfortsätze 44, 44'. Die Anschläge 50, 50' begrenzen jeweils einen Verschwenkwinkel des Griffelements 18. Das Griffelement 18 kann aus der Ausgangslage, insbesondere von einem Bediener, um zumindest annähernd 90° um die Schwenkachse 26 verschwenkt werden, bis das Griffelement 18 an den Anschlägen 50, 50' anliegt. Die 90°-Verschwenkstellung des Griffelements 18 bildet insbesondere eine Tragelage des Griffelements 18 aus, in welcher der Sterilbehälter 12 mittels der Sterilbehälterhaltevorrichtung 10, 10' gehalten werden kann. Unabhängig von einer Lage des Sterilbehälters 12 werden die Griffelemente 18 bei einer fehlenden Belastung durch den Bediener mittels der Rückstellelemente 20, 20' selbsttätig zurück in die Ausgangslage verschwenkt.

Ferner weist der Sterilbehälter 12 eine Verschlussvorrichtung 52 auf. Die Verschlussvorrichtung 52 ist zumindest teilweise auf dem Grundkörper 14 der Sterilbehälterhaltevorrichtung 10 angeordnet. Die Verschlussvorrichtung 52 für den Sterilbehälter 12 umfasst eine schwenkbar gelagerte Verschlussklappe 54, ein zumindest in einer Schließstellung mit der Verschlussklappe 54, insbesondere form- und/oder kraftschlüssig, zusammenwirkendes Rastelement 56, und zumindest eine zumindest eine Befestigungsschnittstelle 58 zu einem Befestigen an dem Sterilbehälter 12 aufweisende Halteeinheit 60 zu einem Halten der, insbesondere schwenkbar an der Halteeinheit 60 gelagerten, Verschlussklappe 54 und/oder des Rastelements 56 an dem Sterilbehälter 12. Bevorzugt ist das Rastelement 56 als ein, insbesondere schwenkbar gelagerter, Spannhebel gemäß der EP 2 559 395 B1 ausgebildet, so dass in Bezug auf die Ausgestaltung des als Spannhebel ausgebildeten Rastelements 56 zumindest im Wesentlichen auf die Offenbarung der EP 2 559 395 B1 verwiesen wird. Es ist jedoch auch denkbar, dass das Rastelement 56 eine andere, einem Fachmann als sinnvoll erscheinende Ausgestaltung, wie beispielsweise eine Ausgestaltung als translatorisch beweglich und federvorgespannter Rastschieber o. dgl., aufweist. Die Halteeinheit 60 der Verschlussvorrichtung 52 ist fest mit dem Grundkörper 14 der Sterilbehälterhaltevorrichtung 10 verbunden.

### Bezugszeichen

- 10: Sterilbehälterhaltevorrichtung
- 12: Sterilbehälter
- 14: Grundkörper
- 16: Sterilbehältergehäuse
- 18: Griffelement
- 20: Rückstellelement
- 22: Lagerausnehmung
- 24: Mittelachse
- 26: Schwenkachse
- 28: Endfortsatz
- 30: Ausnehmung
- 32: Endfortsatz
- 34: Ausnehmung
- 36: Griffbereich
- 38: Behälterdeckel
- 40: Seitenwand
- 42: Seitenwand
- 44: Befestigungsfortsatz
- 46: Ende
- 48: Kragbereich
- 50: Anschlag
- 52: Verschlussvorrichtung
- 54: Verschlussklappe
- 56: Rastelement
- 58: Befestigungsschnittstelle
- 60: Halteeinheit

## Patentansprüche

1. Sterilbehälterhaltevorrichtung für einen Sterilbehälter (12), mit zumindest einem Grundkörper (14) zu einer festen Verbindung mit einem Sterilbehältergehäuse (16) des Sterilbehälters (12) und mit zumindest einem relativ zu dem Grundkörper (14) schwenkbar gelagerten Griffelement (18),
**gekennzeichnet durch**
zumindest ein mit dem zumindest einen Griffelement (18) gekoppelten Rückstellelement (20, 20'), das dazu vorgesehen ist, das Griffelement (18) in zumindest einer Relativstellung zu dem Grundkörper (14) mit einer Kraft zu beaufschlagen.

2. Sterilbehälterhaltevorrichtung nach Anspruch 1,
**dadurch gekennzeichnet, dass**
das Rückstellelement (20, 20') dazu vorgesehen ist, das Griffelement (18) mit einer Kraft in Richtung einer Ausgangslage des Griffelements (18) zu beaufschlagen.

3. Sterilbehälterhaltevorrichtung nach einem der vorhergehenden Ansprüche,
**dadurch gekennzeichnet, dass**
das Rückstellelement (20, 20') als eine Feder, insbesondere eine Schraubenfeder, ausgebildet ist.

4. Sterilbehälterhaltevorrichtung zumindest nach Anspruch 3,
**gekennzeichnet durch**
zumindest eine Lagerausnehmung (22, 22') zur beweglichen Lagerung des Griffelements (18), welche zumindest im Wesentlichen von dem Grundkörper (14) begrenzt ist.

5. Sterilbehälterhaltevorrichtung nach Anspruch 4,
**dadurch gekennzeichnet, dass**
das Rückstellelement (20, 20') zumindest im Wesentlichen in der Lagerausnehmung (22, 22') angeordnet ist.

6. Sterilbehälterhaltevorrichtung nach einem der vorhergehenden Ansprüche,
**dadurch gekennzeichnet, dass**
eine Mittelachse (24) des Rückstellelements (20, 20') zumindest im Wesentlichen parallel zu einer Schwenkachse (26) des Griffelements (18) angeordnet ist.

7. Sterilbehälterhaltevorrichtung nach einem der vorhergehenden Ansprüche,
**dadurch gekennzeichnet, dass**
das Rückstellelement (20, 20') zumindest einen ersten Endfortsatz (28), welcher dazu vorgesehen ist, in eine Ausnehmung (30) des Griffelements (18) einzugreifen, und zumindest einen zweiten Endfortsatz (32) aufweist, welcher dazu vorgesehen ist, in eine Ausnehmung (34) des Grundkörpers (14) einzugreifen.

8. Sterilbehälter mit zumindest einem Sterilbehältergehäuse (16), und mit zumindest einer an dem Sterilbehältergehäuse (16) angeordneten Sterilbehälterhaltevorrichtung (10, 10') nach einem der vorhergehenden Ansprüche.

9. Sterilbehälter nach Anspruch 8,
**dadurch gekennzeichnet, dass**
das Griffelement (18) der Sterilbehälterhaltevorrichtung (10, 10') zumindest einen Griffbereich (36) aufweist, der in einer Ausgangslage des Griffelements (18) an dem Sterilbehältergehäuse (16) anliegt.

10. Sterilbehälter nach Anspruch 8 oder 9,
**dadurch gekennzeichnet, dass**
das Griffelement (18) in einer Ausgangslage des Griffelements (18) in dem Sterilbehältergehäuse (16) eingebettet ist.

11. Sterilbehälter nach einem der Ansprüche 8 bis 10,
**dadurch gekennzeichnet, dass**
das Rückstellelement (20, 20') zumindest im Wesentlichen vollständig von dem Sterilbehältergehäuse (16) und dem Grundkörper (14) der Sterilbehälterhaltevorrichtung (10, 10') umschlossen ist.

12. Sterilbehälter nach einem der Ansprüche 8 bis 11,
**gekennzeichnet durch**
eine weitere Sterilbehälterhaltevorrichtung (10'), insbesondere nach einem der vorhergehenden Ansprüche, wobei die Sterilbehälterhaltevorrichtung (10) und die weitere Sterilbehälterhaltevorrichtung (10') an voneinander abgewandten Seiten des Sterilbehältergehäuses (16) angeordnet sind.
